# EUROPÄISCHE PATENTSCHRIFT

(11) **EP 1 504 730 B1**
(45) Veröffentlichungstag und Bekanntmachung des Hinweises auf die Patenterteilung: **30.05.2007**
(21) Anmeldenummer: 04001535.6
(22) Anmeldetag: 24.01.2004
(51) Int. Cl.: A61F 2/14, A61F 2/16

(54) **Akkommodative Intraokular-Mehrfachlinse**
Accomodative intraocular composite lens
Lentille intraoculaire accommodative composée

(30) Priorität: 06.08.2003 DE 10335973
(43) Veröffentlichungstag der Anmeldung: 09.02.2005
(73) Patentinhaber: Boehm, Hans-Georg, Dr. rer. nat., D-61476 Kronberg/Ts (DE)
(72) Erfinder: Boehm, Hans-Georg, Dr. rer. nat., D-61476 Kronberg/Ts (DE)

(56) Entgegenhaltungen:
- EP-A- 0 356 050
- FR-A- 2 666 735
- US-A- 4 892 543
- US-A- 5 489 302

## Beschreibung

### Stand der Technik :

Bisher bekannte akkommodative einteilige Intraokularlinsen IOL, die zur Behebung des grauen Stars (Linsentrübung) statt der natürlichen Linse in den Kapselsack des Auges implantiert werden, ermöglichen eine eingeschränkte Akkommodation (bis maximal 1,5 dpt), wenn sie sich bei Kontraktion oder Entspannung des Ziliarmuskels durch geeignet geformte Zentrierstege axial verschieben (US 6,485,516 B2). Die Fähigkeit zur Axialverschiebung verliert sich aber postoperativ im Laufe der Zeit, weil der Kapselsack dann unvermeidlich schrumpft.

Aus der Literatur (HARA, T. et al.:Accommodative Intraocular Lens with Spring Action Part 1. In: Opthalmic Surgery 1990, 21 S. 128-133) ist ein implantierbares Linsensystem bestehend aus zwei Linsen bekannt. Eine solche Doppellinse akkommodiert, wenn ihr Fokus durch das Wirken des Ziliarmuskel in ähnlicher Weise beeinflußt wird, wie es die Natur vormacht. Dort wölbt sich bei angespanntem Ziliarmuskel bzw. bei entspannten Zonulafasern die körpereigene Linse kugelförmig für den Nahfokus oder flacht sich bei entspanntem Ziliarmuskel bzw. bei angespannten Zonulafasern für den Fernfokus ab.

Die Akkommodation einer IOML kann durch eine mit der Hauptlinse kombinierte, dünne Korrekturlinse mit federndem Seitenrand wirkungsvoll und langanhaltend verbessert werden, wenn sich zwischen beiden Linsen Luft befindet.

Dazu ist die Patentanmeldung EP-A-0 356 050 (STORZ INSTR CO) vom 28. Februar 1990 (1990-02-28) als nächstliegenden Stand der Technik anzusehen. Dort ist der von zwei halbkugelförmigen, flexiblen Linsen eingeschlossene Hohlraum flüssigkeitsgefüllt. Weitere Patentanmeldungen US 4 932 966, US 4 842 601, US 5 152 788 A wurden berücksichtigt.

### Problemlösung und Beschreibung :

Diese Aufgabe wird durch eine akkommodative Intraokular-Mehrfachlinse (IOML) gemäß Anspruch 1 gelöst. Ein weiteres Merkmale ist im Anspruch 2 aufgenommen.

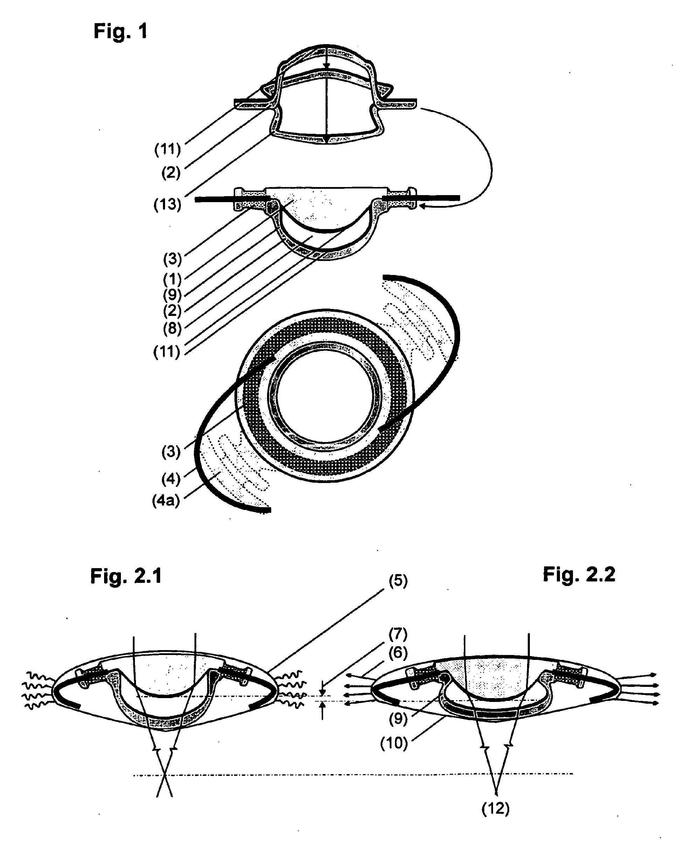

Fig. 1: zeigt eine akkommodative IOML mit Zentrierfedern (4), bestehend aus einer Hauptlinse (1) bspw. aus hydrophilem Acrylat mit festem Grundfokus und einer dünnen Korrekturlinse (2) mit federndem Seitenrand (9), die zur Änderung des Gesamtfokus (12) des Systems herangezogen wird. Die halbkugelförmige Korrekturlinse ist dabei axial und im optisch wirksamen Bereich berührungslos über die Hauptlinse gestülpt und am äußeren Rand luftdicht mit ihr verbunden (3). Weil gebräuchliche Linsenmaterialien feuchtigkeitshaltig sind, sind die Innenseiten des luftgefüllten Hohlraums (8) zwischen den beiden Linsen feuchtigkeitsundurchlässig beschichtet (11), um eine Diffusion von Feuchtigkeit dort hinein, Schlierenbildung, Reflexionen und gegenseitiges Anhaften zu vermeiden. Zur Stärkung der Rückstellkraft des federnden Seitenrandes (9) durch Memoryeffekt ist die Korrekturlinse nach der Beschichtung, aber vor ihrem Zusammenfügen mit der Hauptlinse, bspw. durch einen Ultraschallschweißrand (3), in sich umgestülpt (13). Die IOML ist zusammengerollt ebenso einfach zu implantieren wie eine einteilige IOL und lässt sich zur Verstärkung ihrer Axialverschiebung auch mit den bekannten Zentrierstegen aus US 6,485,516 B2 kombinieren (4a).
Fig. 2.1: zeigt eine implantierte IOML bei entspannten Zonulafasern. Durch die Rückstellkraft der Korrekturlinse ist hier der Kapselsack axial gedehnt (5), unterstützt durch das Luftpolster im Hohlraum, das wie ein flexibler Puffer wirkt. So werden dessen Membrane unter Spannung gehalten und sein Volumen vorteilhaft ausgefüllt, um seiner postoperativen Schrumpfung entgegen zu wirken. Die beiden Linsen bestehen aus unterschiedlich flexiblem, körperfreundlichem Kunststoff, wobei die Korrekturlinse aus stärker federndem Material besteht, um sich stets für den Nahfokus in ihre halbkugelförmige Ausgangsform zurück zu bilden und dabei zusätzlich die Hauptlinse zur Axialverschiebung in Richtung von der Retina weg hin zur vorderen Kapselsackmembran zu drücken (7).
Fig. 2.2: zeigt den abgeflachten Kapselsack (10) bei angespannten Zonulafasern (6). Für den Weitfokus wird hier die Korrekturlinse näher an die Hauptlinse gedrückt und vergrößert so die Brennweite des Linsensystems (12). Gleichzeitig wird die Hauptlinse etwas axial zur Retina hin verschoben und unterstützt zusätzlich diese Fokusänderung (7).

Beim Quetschen des Seitenrandes (9) der Korrekturlinse ändert sich wirkungsvoll die Brechkraft des zwischen den beiden Linsen liegenden Hohlraums, weil die IOML in Kapselsackflüssigkeit mit hohem Brechungsindex eingebettet ist, er aber Luft von Brechungsindex = 1 enthält. Die IOML reagiert daher empfindlicher und fast ohne Hysterese trotz der vergleichsweise geringen Änderung der Kapselsackform als dies bei alleiniger Axialverschiebung einer einteiligen Linse der Fall ist. Die Korrekturlinse selbst ist zu dünn, um den Strahlengang nennenswert zu beeinflussen.

### Bezeichnungen :

(1) Hauptlinse für den Grundfokus, der den leicht negativ wirkenden Fokus der Korrekturlinse (2) überkompensiert; ihre Krümmung ist vom Brechungsindex des verwendeten Linsenmaterials abhängig
(2) dünne Korrekturlinse mit federnd flexiblem Seitenrand (9)
(3) angedeuteter Ultraschallschweißrand, der die beiden Linsen (1+2) luftdicht verschließt
(4) Zentrierfedern; dünn, zur Erleichterung der Implantation oder (4a) ersetzt durch die aus US 8485516 bekannten Zentrierstege
(5) durch die kugelförmige Korrekturlinse gedehnter und durch deren ständige Bewegung trainierter Kapselsack
(6) Zonulafasern, die den Ziliarmuskel mit dem Kapselsack verbinden
(7) Axialverschiebung der IOML
(8) Hohlraum, feuchtigkeitsundurchlässig beschichtet (11), enthält Luft für hohe Änderung des Brechungsindex, was entscheidend für die beeinflussbare Fokusänderung ist
(9) federnder, hier gequetschter Rand der Korrekturlinse (2) bei
(10) abgeflachtem Kapselsack
(11) beschichtete Innenseiten des Hohlraums (8)
(12) Strahlengang und Fokus, verkürzt gezeichnet, zeigt in Richtung der Retina
(13) nach der Oberflächenbeschichtung (11) ist die Korrekturlinse in sich umgestülpt, um den Memoryeffekt für eine verbesserte Rückstellkraft ihres Seitenrandes (9) zu nutzen

## Patentansprüche

1. Akkommodative Intraokular-Mehrfachlinse (IOML) als Ersatz für getrübte körpereigene Augenlinsen bestehend aus einer Hauptlinse (1) und einer dünnen Korrekturlinse (2)mit einem dünnen flexiblen Seitenrand (9), die halbkugelförmig und axial ohne sich im optischen Bereich zu berühren über die Hauptlinse gestülpt ist und an ihrem Rand (3) luftdicht mit ihr verbunden ist und sich im Hohlraum (8) Luft befindet, ist **dadurch gekennzeichnet, daß** die Innenseiten des Hohlraums feuchtigkeitsundurchlässig, reflexionsmindernd und antihaftend beschichtet sind.

2. Akkommodative IOML ist **dadurch gekennzeichnet, daß** der dünne, flexible Rand der Korrekturlinse (9) eine verstärkte Rückstellkraft aufweist als es dem normalem Linsenmaterial entspricht und dadurch erzielt ist, daß die Korrekturlinse (2) vor dem Zusammenfügen (3) mit der Hauptlinse in sich umgestülpt ist.

## Claims

1. Accommodating intraocular lens (IOML) for replacement of muddy natural eye lenses consisting of a main lens (1) and a thin correction lens (2) with a thin flexible margin (9) which is put hemispherically and axially over the main lens without touching this one and is airtightly connected to the main lens around its border (3) and contains air in the cavity (8) having the insides coated impenetrably to humidity, reflection reduced and non adhering.

2. Accommodating IOML with a thin flexible margin (9) of its correction lens having an amplified repelling force as compared to the normal lens material by turning the correction lens (2) upside down before connecting it (3) to the main lens.

## Revendications

1. Lentille intraoculaire accommodative (IOML) comme remplacement de lentilles naturelles troubles qui consiste d'une une lentille principale (1) et d'une lentille mince de correction (2) avec une marge flexible et mince (9) dont laquelle est mis hémisphérique et axiale sur la lentille principale sans toucher celle-ci et est connectée hermétique à la lentille principale autour de son bord (3) et contient de l'air dans la cavité (8), qui possède à sa face intérieure une couche impénétrable à humidité, réflexion réduite et non adhérente.

2. Lentille IOML avec une marge flexible et mince (9) de sa lentille de correction qui possède une force repoussante amplifiée comparée à la matière d'une lentille normale en tournant la lentille de correction (2) sens dessus dessous avant de la connecter (3) à la lentille principale.
